# EUROPEAN PATENT APPLICATION

(11) **EP 2 196 094 A1**
(43) Date of publication of application: **16.06.2010**
(21) Application number: 07825162.6
(22) Date of filing: 21.09.2007
(51) Int. Cl.: A23D 7/015, A23D 9/00, C11C 3/10

(54) **FATTY PRODUCT WITH LOW QUANTITY OF SATURATED FAT AND BASICALLY COMPOSED OF STEARIC ACID**

(71) Applicant: Aceites y Grasas Vegetales S.A. - Acegrasas S.A., Autopista Sur No. 57-21 1 Bogóta (CO)
(72) Inventor: RODRIGUEZ POSADA, Luidy Alfonso, 1 Bogóta (CO); CRUZ SERNA, Adriana Fernanda, 1 Bogóta (CO)
(74) Representative: Matthews, Derek Peter
(86) International application number: PCT/IB2007/002755
(87) International publication number: WO 2009/037521

(57) **Abstract**

The present invention concerns a fatty product which contains a low concentration of saturated fatty acids. These saturated fatty acids are mainly composed of stearic fatty acid and have a very low percentage of palmitic acid. This fatty product is obtained from intersterification of: (1) 10 to 80% of a totally hydrogenated vegetable oil with (2) 20 to 90% of a liquid vegetable oil having a very low content in saturated fatty acids. The fatty product of the present invention can be used directly as an ingredient for preparing breadmaking products, such as tarts, biscuits and loaves among others, and indirectly for making margarines.

## Description

### TECHNICAL FIELD

The present invention concerns a fatty product containing a low amount of saturated fatty acids. These saturated fatty acids are mainly composed of stearic fatty acid.

The fatty product of the present invention can be directly used as an ingredient for manufacturing bread products, such as tarts, biscuits and loaves among others, and indirectly for making margarines.

### BACKGROUND OF THE INVENTION

It has been found that high cholesterol levels in the blood serum are related with a high risk of generating heart coronary diseases (HCD), which as is well known, show different clinic manifestations which may go from an angina pectoris to myocardial infarction and sudden death.

The associations between cholesterol serum levels and HCD are directly related with high levels of low density lipoproteins (LDL), which are the main carriers of cholesterol through blood stream.

Various studies¹ have proven that quantity and composition of food fats are the main determinants of cholesterol levels in serum. Also, it has been found a direct relation between the consumption of saturated fatty acids and cholesterol with cholesterol in serum. Furthermore, it has been demonstrated that polyunsaturated fatty acids show a reducing effect of LDL cholesterol level in serum.
¹ HEGSTED, D.M. et al. "Quantitative effects of dietary fat on serum cholesterol in man". American Journal of Clinical Nutrition, 17: 281-295, 1965.

Clinic studies² suggest that various saturated fatty acids show several effects in serum cholesterol levels. In general, data indicates that stearic acid in the majority of natural fats has low influence on serum cholesterol levels, whereas myristic acid, among saturated fatty acids, increases the most the cholesterol level. Nevertheless, palmitic acid is the main saturated acid in the great majority of diets.
² KEYS, A.; ANDERSON, J.T. and GRANDE, F. "Serum cholesterol response to changes in the diet. IV. Particular saturated fatty acids on the diet". Metabolism 14: 776-787, 1965

Palmitic (16:0), lauric (12:0) and myristic (14:0) acids are considered the main fatty acids producing hypercholesterolemia. Nevertheless, each one of these fatty acids produces a different effect on cholesterol level. The following figure summarizes the recent results concerning the fatty acids effect on serum cholesterol levels.

Figure 1. fatty acids effects on serum cholesterol levels³

³ KATAN, M.B., ZOCK, P.L. and MENZINK, R.P. "Efectos de los ácidos grasos individuales sobre el colesterol sérico total y el de las lipoproteínas (datos procedentes de varias fuentes)". Grasas y Lipoproteinas Séricas, 1993

.

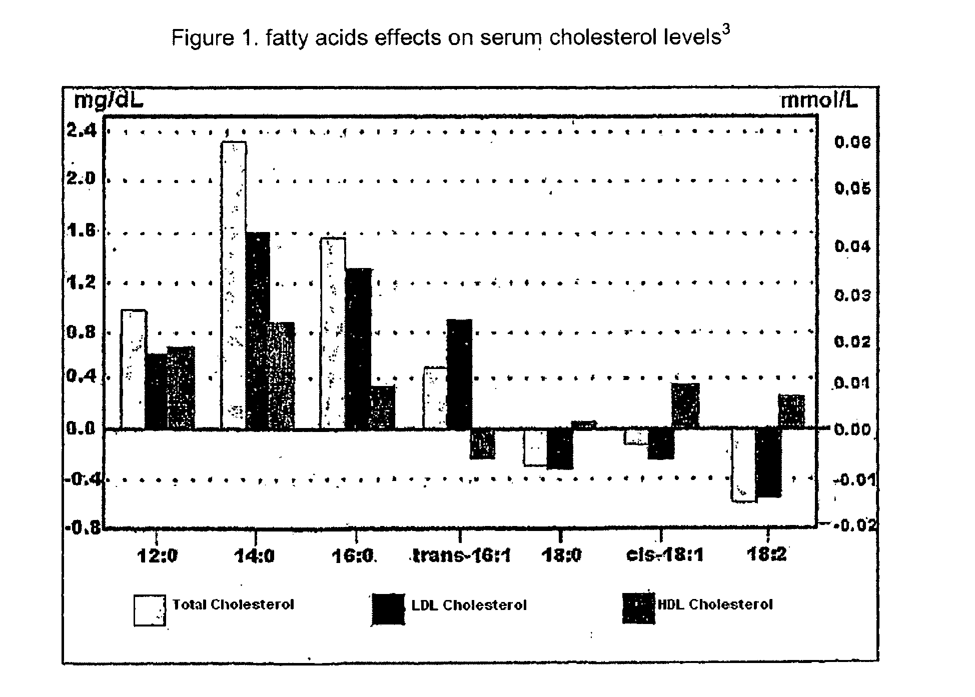

On the other hand, it has been found a strong relationship between trans fatty acids and HCD development. Trans fatty acids affect the cholesterol profile because they increase the total cholesterol, as well as LDL and reduce the high density lipoproteins (HDL).

HDL is a kind of lipoprotein which eliminates cholesterol from arteries and transports it to liver for excretion, this is way HDL is known as good cholesterol.
HDL cholesterol poses no threat to cardiovascular health, opposed to LDL or bad cholesterol.

Due to the above factors, there is the need for developing fatty products containing low levels of: cholesterol, trans fatty acids and saturated fatty acids. Furthermore, saturated fatty acids should be balanced in such way so that the greater proportion corresponds to stearic acid. Taking the fatty products, which are a fundamental part of world population diet, to the above-mentioned standards, will contribute to reduce the risk of obtaining HCD.

There have been much progress in this area in the last years, but, there is still the need for producing fats which contribute even more with controlling blood cholesterol.

Patent US 5,215,779, refers to producing a multipurpose lard having low content of saturates fatty acids (less than 20%), which contains a partially hydrogenated component. This component delivers medium melting point triglycerides which grants it with a good plasticity. Nevertheless, this component has a high trans fatty acids level, which is unwanted due to its relationship with the risk of getting HCD.

US patent No 5,407,695 discloses a margarine and a lard having reduced levels of trans fatty acids and palmitic fatty acid. The product is obtained through a mixture of liquid oil with a completely hydrogenated fat, in which almost 15% of palmitic fatty acid was replaced by short chain acids such as: acetic, propionic and butyric. However, short chain saturated fatty acids are easily hydrolysable turning the product unstable in the presence of water. Short chain acids are perceptible to the palate even at very low concentrations. These fatty acids have flavor notes which go from rancid to soapy.

### ABSTRACT OF THE INVENTION

The present invention relates to developing a fatty product containing a low amount of saturated fatty acids. These saturated fatty acids are mainly composed of stearic fatty acid.

The fatty product of the invention is obtained from intersterification of : (1) a totally hydrogenated vegetable oil with (2) a liquid vegetable oil having a very low content of saturated fatty acids.

The fatty product of the present invention can be used directly as an ingredient for preparing bread products, such as tarts, biscuits and loaves among others, and indirectly for making margarines.

Additional objectives and advantages of the invention will be more apparent after reading the following detailed description and claims.

### DETAILED DESCRIPTION OF THE INVENTION

The purpose of present invention is obtaining a fatty product containing a low amount of saturated fat, a good proportion of stearic fatty acid and a low level of trans fatty acids. This fatty product must have excellent functional and organoleptic properties which allow its direct application as ingredient in bread industry and indirectly for preparing other fatty products such as margarines.

The main characteristics of the fatty product of the present invention are the content of less than 45% total saturated fatty acids, a percentage of stearic fatty acid between 20 and 30% and palmitic fatty acid between 5 and 15%, and a very low level of trans fatty acids.

A low amount of saturated fatty acids such as stearic fatty acid and trans fatty acids make the food elaborated with the fatty product of the invention, healthier, due to the fact that the intake of such food reduce the risk of getting HCD.

The background of the invention explained that palmitic acid has a relationship with serum cholesterol increase; therefore, it is desirable that the fatty product of the present invention has a low level of this fatty acid. Nevertheless, in order that the fatty product possesses the required functional properties to be used for preparing bread products and margarine, there must be some amount of palmitic fatty acid, which contributes to have fatty products with type β' stability.

The vegetable fats are polymorphic, that is, are capable of forming different types of crystalline structures during their crystallization process. The main types of crystals which can be formed are: α (alpha), β' (Beta prime) and β (beta). α crystals, which are the smallest, have a smooth structure but are very unstable. β' crystals have a medium size and are more wanted for elaborating fatty products and margarines because they provide a smooth texture, they are stable and secure the plasticity of the final product. The bigger crystals are type β, which are stable but in general unwanted due to their granular texture and that are easily converted in a stiff and breakable structure.

In order to elaborate fatty products having an improved β' stability, the following factors⁴ must be taken into account: a) a good palmitic fatty acid content, b) an appropriate distribution and position of palmitic and stearic fatty acids within the triglyceride molecule, c) certain degree of hydrogenation and d) a good randomization degree, possible to have in natural form or through a intersterification process.
⁴ O'BRIEN, Richard. Fats and Oils. Formulating and Processing for Applications, CRC Press, Second edition, 2004

The fatty product of the invention is obtained from intersterification of : (1) a totally hydrogenated vegetable oil with (2) a liquid vegetable oil having a very low content in saturated fatty acids.

### (1) Totally hydrogenated vegetable oil

In order to obtain a totally hydrogenated vegetable oil, one must start with a vegetable oil containing a palmitic fatty acid concentration less than 20%, preferably less than 15%, and a 18 carbon fatty acid (stearic, oleic, linoleic, and/or linolenic acids) content greater than 70%, preferably greater than 85%.

Among the oils which comply with these characteristics we can find sunflower, sunflower having high oleic content, NuSun^{™} sunflower, canola, olive, corn, safflower, safflower having high oleic content, peanut, linseed and rice, among others.

In order to obtain the totally hydrogenated vegetable oil, the hydrogenation process can be carried out through batch or continuous process. There are two types of batch process which are: hydrogenation in systems having recirculation and hydrogenation in dead-end systems⁵.
⁵ HUI, Y.H. Bailey's Industrial Oil and Fat Products- Volume 4. John Wiley & Sons, Inc. Fifth Edition, 1996

The general purpose of hydrogenation is the saturation of double bonds of the unsaturated fatty acids with hydrogen. During the process, several chemical changes take place such as geometric isomerization, positional isomerization, conjugation and the hydrogenation. The catalytic addition of hydrogen to the double bonds in the fatty acids chains provides an effective means for modifying the properties of oils and fats. The reaction product is a saturated compound. During hydrogenation process, the linolenic fatty acid is the first one to be hydrogenated producing linoleic fatty acid. Then, the linoleic acid is hydrogenised producing oleic acid, which is finally hydrogenated producing stearic fatty acid.

In general, the hydrogenation process is carried out in the presence of catalysts mainly based on nickel. The factors influencing on the hydrogenation process and on the characteristics of the products resulting from such process are: temperature, pressure, stirring and even the nature and amount of the catalyst used.

### (2) Liquid vegetable oil having a very low content in saturated fatty acids.

The liquid vegetable oil must have a concentration less than 20% of saturated fatty acids, preferably less than 15%.

The liquid vegetable oil having a low content of saturated fatty acids can be chosen among sunflower, sunflower having high oleic content, NuSun^{™} sunflower, canola, safflower, safflower having high oleic content, corn, linseed oils, etc. or can be a mixture of said oils.

### Intersterification of (1) totally hydrogenated oil with (2) a liquid vegetable oil having a very low content in saturated fatty acids

The intersterification is characterized by reacting two fatty materials aided by a catalyst for obtaining a new fatty material, which has a fatty acids distribution in its triglycerides different to that of the original two fatty materials. By altering the distribution of fatty acids in the triglycerides, the melting and crystallization characteristics differ from the mixture of the original fatty materials.

There are different types of intersterification among which there is the chemical intersterification and the enzymatic intersterification.

The chemical intersterification can be random or directed. In the random intersterification, the molecules of the fatty acids are randomly distributed in the triglyceride molecules obeying the probability laws. In the directed intersterification, which is done at low temperature, a portion of the mixture being intersterificated in liquid state crystallizes such that the reactive mass is constantly changing in composition and generating more material which crystallizes. Accordingly, the directed intersterification allows to obtain triglycerides having a very high melting point or very low melting point.

The catalysts for chemical intersterification commercially used include: sodium methoxide, sodium and potassium alloys and potassium or sodium hydroxide.

The enzymatic intersterification is carried out by using enzymatic catalysts, which allows a greater control of the reaction and a better specificity with respect to chemical intersterification. There are two types of enzymatic catalysts in the market: lipases which produce similar products to those of chemical intersterification and lipases which have a specificity for positions 1 and 3 of the triglycerides which allow the production of specific triglycerides in high concentration.

The present invention encompasses a random chemical or enzymatic intersterification of (1) 10 to 80% of a totally hydrogenated vegetable oil with (2) 20 to 90% of a liquid vegetable oil having a very low content in saturated fatty acids; preferably, a random chemical or enzymatic intersterification of (1) 20 to 50% of a totally hydrogenated vegetable oil with (2) 50 to 80% of a liquid vegetable oil having a very low content in saturated fatty acids.

The fatty product resulting from the intersterification shows a percentage of total saturated fatty acids lower than 45%, and preferably lower than 36%.

In an embodiment of the invention, it is obtained a fatty product wherein the percentage of stearic fatty acid is around 20 to 30%, preferably between 24 and 27%; while the percentage of palmitic fatty acid is around 5 to 15%, preferably between 7 and 10%.

It has been found that the product of the invention is very adequate to be used as fatty raw material for elaborating food products such as cookies, tarts and other bread products as well as for elaborating margarines and spreadable products.

### Example 1:

Bleached and deodorized, refined soyseed oil, having the following fatty acids profile was loaded, obtained from a gas chromatogram:

| Fatty acid | Percentage |
|---|---|
| Myristic | 0.1 |
| Palmitic | 12.7 |
| Stearic | 4.3 |
| Oleic | 21.1 |
| Linoleic | 53.7 |
| Linolenic | 8.1 |
| Total saturated fatty acids | 17.1 |
| Total monounsaturated fatty acids | 21.1 |
| Total polyunsaturated fatty acids | 61.8 |

was loaded in a hydrogenation tank and was heated up to 150°C using a heating jacket with steam and under vacuum in order to eliminate humidity. An amount of 300 ppm of nickel catalyst was then added for non-selective hydrogenation. Hydrogen addition started until an inner pressure of 200 manometric kPa (30 psig) was obtained in the tank. The temperature was increased up to 180-200°C due to the hydrogenation reaction and the pressure was held at 200 manometric kPa (30 psig). The hydrogenation reaction took about 24 hours. The iodine index of soybean, which initially was 127 g I₂/100g sample, was reduced down to a quantity lower than 1 g I₂/100g sample. The hydrogen addition valve was then closed, and proceeded with cooling the product using cold water through the jacket.

The completely hydrogenated soybean was then again refined, bleached and deodorized and showed the following fatty acids profile:

| Fatty acid | Percentage |
|---|---|
| Myristic | 0.3 |
| Palmitic | 13.4 |
| Stearic | 83.9 |
| Oleic . | 2.1 |
| Linoleic | 0.3 |
| Total saturated fatty acids | 97.6 |
| Total monounsaturated fatty acids | 2.1 |
| Total polyunsaturated fatty acids | 0.3 |

30% of the completely hydrogenated, refined, bleached and deodorized soybean, was mixed with 70 % of sunflower refined, bleached and deodorized oleic, having a high oleic content, had the following fatty acids profile:

| Fatty acid | Percentage |
|---|---|
| Myristic | 0.1 |
| Palmitic | 4.7 |
| Stearic | 3.0 |
| Oleic | 82.8 |
| Linoleic | 9.0 |
| Linolenic | 0.4 |
| Total saturated fatty acids | 7.8 |
| Total monounsaturated fatty acids | 82.8 |
| Total polyunsaturated fatty acids | 9.4 |

The product of the mixture has the following fatty acids profile:

| Fatty acid | Percentage |
|---|---|
| Myristic | 0.2 |
| Palmitic | 7.3 |
| Stearic | 27.3 |
| Oleic | 58.6 |
| Linoleic | 6.4 |
| Linolenic | 0.3 |
| Total saturated fatty acids | 34.7 |
| Total monounsaturated fatty acids | 58.6 |
| Total polyunsaturated fatty acids | 6.7 |

The mixture was heated under vacuum conditions up to 105°C. 0.1% of sodium methoxide was added as catalyst and the intersterification reaction started and took around 5 min. The catalyst was withdrawn through a filter press. The product resulting from the intersterification was refined, bleached and deodorized.

The following table summarizes the results of the intersterification process:

| Parameter | | Before intersterification | After intersterification |
|---|---|---|---|
| Melting point (°C) | | 59.2 | 40.3 |
| Solids 10°C | (SFC*) | 32.68 | 17.49 |
| | 20°C | 31.71 | 11.94 |
| | 30°C | 29.67 | 6.12 |
| | 40°C | 26.03 | 3.74 |

| | | | |
|---|---|---|---|
| *SFC: Solid Fat Content | | | |

The resulting fatty product showed an oxidative stability OSI (Oxidative Stability Index) at 110°C for 60 hours. The product of this example was used as ingredient for elaborating cookie mass.

It will be evident to a skilled in the art that various substitutions and modifications can be made to the herein described invention, without departing from the scope and spirit of the invention. Thus, it must be understood that even if the present invention has been illustrated through specific embodiments and optional characteristics; modifications and variations of the concepts herein described can be done by a skilled in the relevant art. Such modifications and variations are considered to be within the scope of this invention.

## Claims

1. A fatty product having a low amount of saturated fat mainly composed of stearic fatty acid and a low percentage of palmitic acid, obtained though intersterification of: (1) 10 to 80% of a totally hydrogenated vegetable oil with (2) 20 to 90% of a liquid vegetable oil having a very low content in saturated fatty acids.

2. The fatty product of claim 1, wherein the intersterification is preferably: (1) 20 to 50% of a totally hydrogenated vegetable oil with (2) 50 to 80% of a liquid vegetable oil having a very low content in saturated fatty acids.

3. The fatty product of claim 1, wherein the saturated fat content is less than 45%.

4. The fatty product of claim 1, wherein the total saturated fat content is preferably less than 36%.

5. The fatty product of claim 1, wherein the percentage of stearic fatty acid is between 20 and 30%.

6. The fatty product of claim 1, wherein the percentage of stearic fatty acid is preferably between 24 and 27%.

7. The fatty product of claim 1, wherein the percentage of palmitic fatty acid is between 5 and 15%.

8. The fatty product of claim 1, wherein the percentage of palmitic fatty acid is preferably between 7 and 10%.

9. The fatty product of claims 1 and 2, wherein in order to obtain the completely hydrogenated vegetable oil, one must start from a vegetable oil having a palmitic fatty acid concentration less than 20% and an 18 carbon fatty acids content greater than 70%.

10. The fatty product of claims 1 and 2, wherein in order to obtain the completely hydrogenated vegetable oil, one must start from a vegetable oil having a palmitic fatty acid concentration less than 15% and an 18 carbon fatty acids content greater than 85%.

11. The fatty product of claims 1 and 2, wherein the liquid vegetable oil must have a saturated fatty acids concentration less than 20%.

12. The fatty product of claims 1 and 2, wherein the liquid vegetable oil must preferably have a saturated fatty acids concentration less than 15%.
